# EUROPEAN PATENT SPECIFICATION

(11) **EP 4 321 103 B1**
(45) Date of publication and mention of the grant of the patent: **14.05.2025**
(21) Application number: 22190118.4
(22) Date of filing: 12.08.2022
(51) Int. Cl.: A61B 6/12, A61B 6/00, A61B 6/50, A61B 90/00

(54) **DYNAMIC VESSEL ROADMAP GUIDANCE**
DYNAMISCHE FÜHRUNG DURCH ANWEDUNG EINES ÜBERBLICK BILDES
GUIDAGE DYNAMIQUE PAR L'APPLICATION D'UNE IMAGE D'ENSEMBLE

(43) Date of publication of application: 14.02.2024
(73) Proprietor: Siemens Healthineers AG, 91301 Forchheim (DE)
(72) Inventor: NARASIMHA MURTHY, Venkatesh, Hillsborough, 08844 (US); VIZITIU, Anamaria, 520038 Sfantu Gheorghe (RO); LIAO, Rui, Princeton Junction, 08550 (US)
(74) Representative: Kraus & Lederer PartGmbB

(56) References cited:
- US-A1- 2008 199 048
- US-A1- 2020 222 018
- HAMMAMI HOUDA ET AL: "Catheter navigation support for liver radioembolization guidance: feasibility of structure-driven intensity-based registration", INTERNATIONAL JOURNAL OF COMPUTER ASSISTED RADIOLOGY AND SURGERY, SPRINGER, DE, vol. 15, no. 11, 1 September 2020 (2020-09-01), pages 1881 - 1894, XP037285942, ISSN: 1861-6410, [retrieved on 20200901], DOI: 10.1007/S11548-020-02250-8

## Description

### TECHNICAL FIELD

The invention generally relates to providing guidance of a catheter in vessels. More precisely, the invention relates to providing dynamic vessel roadmaps to guide a catheter to a vessel of interest.

### BACKGROUND

Medical interventions in vessels to treat pathological vessel conditions, such as obstructions, typically rely on a catheter or some other means. Depending on the specific pathological vessel to be treated, the catheter is inserted into the vessels, e.g. starting at an arm or in the groin area and advanced via major vessels to reach the general vicinity of the pathological vessel. Since vessels tend to branch extensively, at least starting in the general vicinity of the pathological vessel a medical specialist or operator relies on imaging methods based on contrast media and radiation, such as fluoroscopy, to determine the path through the vessel to reach the pathological vessel. Depending on the position of the pathological vessel in the vessel tree and the skill of the medical specialist, a patient may be exposed to high doses of both radiation and contrast media during the medical intervention. Given the negative effects of such doses on the human body, shortening the time needed to reach the pathological vessel and thereby reducing the doses of radiation and of contrast medium would be beneficial. Therefore, it is an objective of the present invention to enable guidance to a pathological vessel.

US2008199048A1 may be considered to disclose a computer-implemented pathological vessel guidance method, comprising the steps of: generating a vessel roadmap library , the vessel roadmap library including a plurality of vessel roadmaps , wherein each vessel roadmap comprises a vessel roadmap image and first and second alignment data ; wherein the method is used by the user to see, within the vessel roadmap images of the vessel roadmap library, a stenosis in a vessel; obtaining a real-time fluoroscopy image and corresponding real-time first and second fluoroscopy information; selecting a vessel roadmap from the roadmap library based on comparing the first real-time fluoroscopy information with the first alignment data of each vessel roadmap of the roadmap library; overlaying the real-time fluoroscopy image with the selected vessel roadmap image of the vessel roadmap library; aligning the vessel roadmap image of the selected vessel roadmap and the real-time fluoroscopy image.

### SUMMARY OF THE INVENTION

To achieve this objective the present invention provides a computer-implemented pathological vessel guidance according to claim 1.

### BRIEF DESCRIPTION OF THE DRAWINGS

Embodiments of the present invention will be described with reference to the following appended drawings, in which like reference signs refer to like elements.
Figs. 1, 1A and 1B show a schematic diagram of a pathological vessel guidance workflow according to embodiments of the invention.
Figs. 2A and 2B illustrate a pathological vessel guidance method according to embodiments of the invention.
Fig. 3 shows examples of segmentations which may be obtained from a vessel image sequence according to embodiments of the invention.
Fig. 4 shows an example of a vessel roadmap according to embodiments of the invention.
Fig. 5 shows an example of a real-time fluoroscopy image and information associated therewith according to embodiments of the invention.
Fig. 6 shows examples of laying a vessel roadmap over a real-time fluoroscopy according to embodiments of the invention.
Fig. 7 shows an example of aligning a vessel roadmap and a real-time fluoroscopy image according to embodiments of the invention.
Figs. 8A and 8B show an exemplary medical imaging system according to embodiments of the invention.
Fig. 9 shows an exemplary computing device according to embodiments of the present invention.

It should be understood that the above-identified drawings are in no way meant to limit the disclosure of the present invention. Rather, these drawings are provided to assist in understanding the invention. The person skilled in the art will readily understand that aspects of the present invention shown in one drawing may be combined with aspects in another drawing or may be omitted without departing from the scope of the present invention.

### DETAILED DESCRIPTION

The invention generally provides a computer-implemented pathological vessel guidance method. The method first generates a vessel roadmap library based on vessel images obtained from a patient using an imaging metho, such as angiography. The vessel images are then processed using image segmentation and to generate the vessel roadmap library. The processing of the vessel images additionally includes detecting one or more pathological vessels. During a subsequent fluoroscopy, the method selects a vessel roadmap from the vessel roadmap library and overlays the corresponding vessel roadmap image over the fluoroscopy image. To guide a fluoroscopy object inserted into the vessels during the fluoroscopy, the method limits the vessels visible in the vessel roadmap library to vessel leading to the detected one or more pathological vessels.

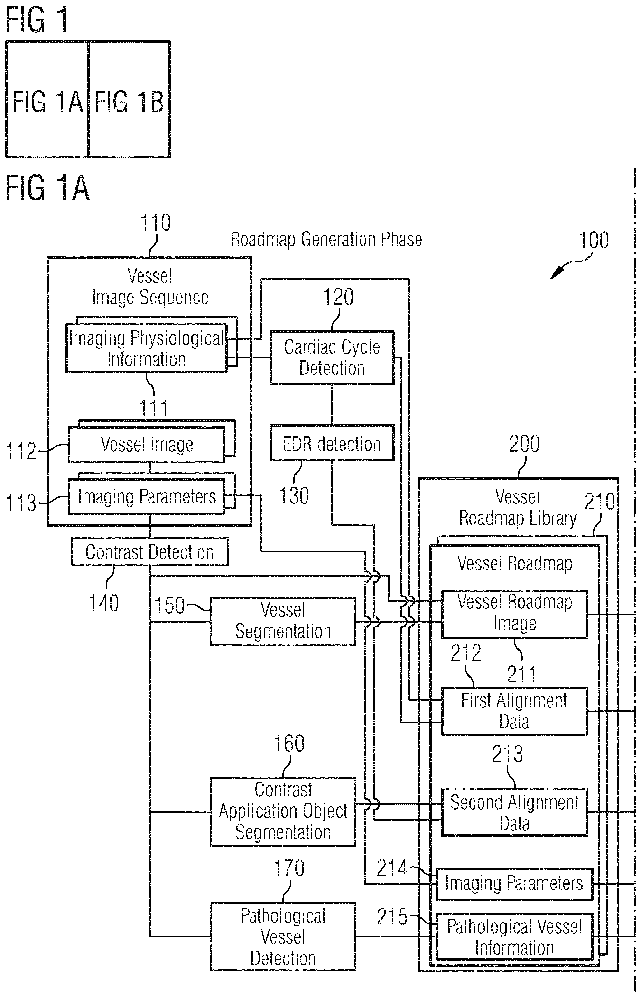

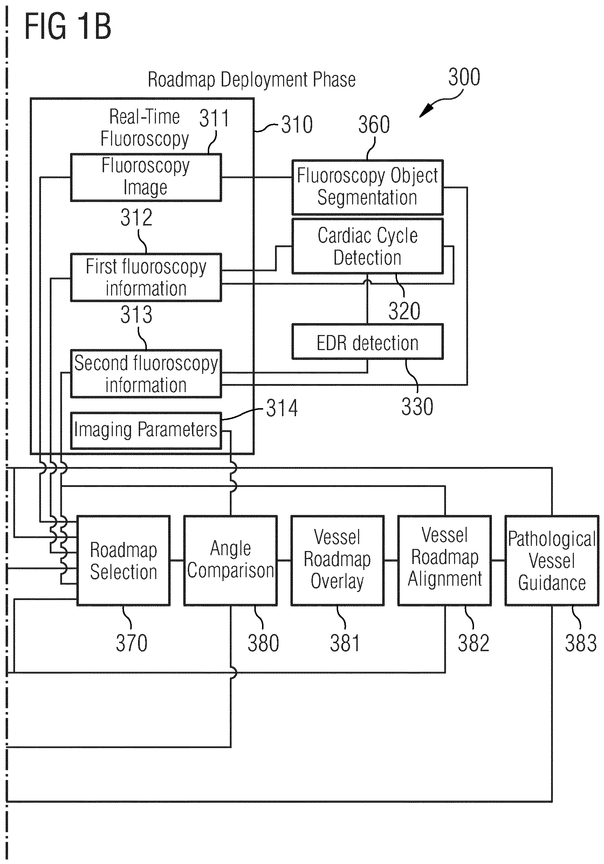

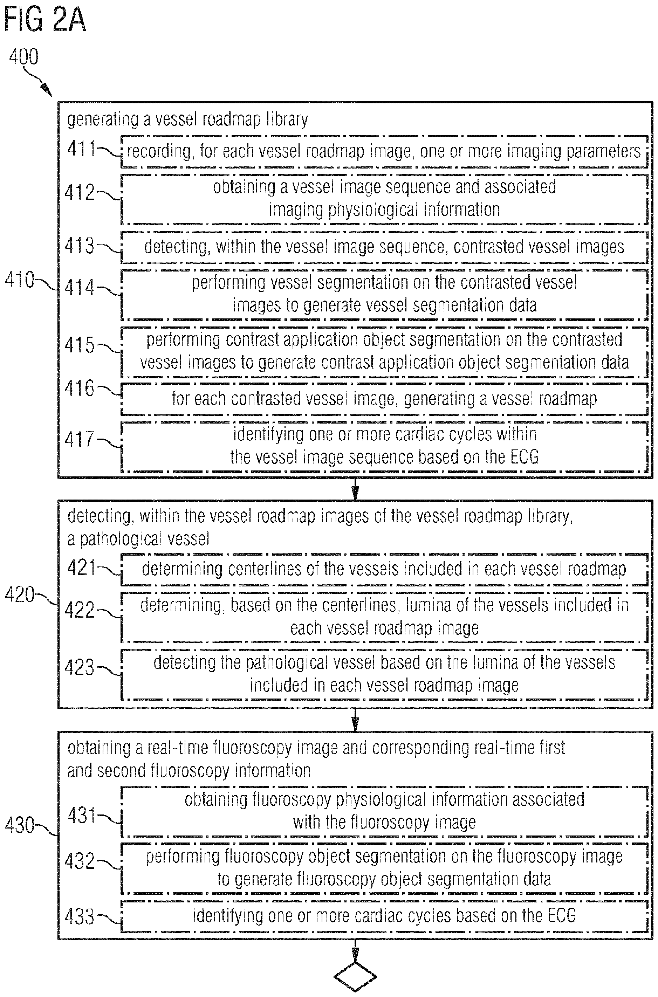

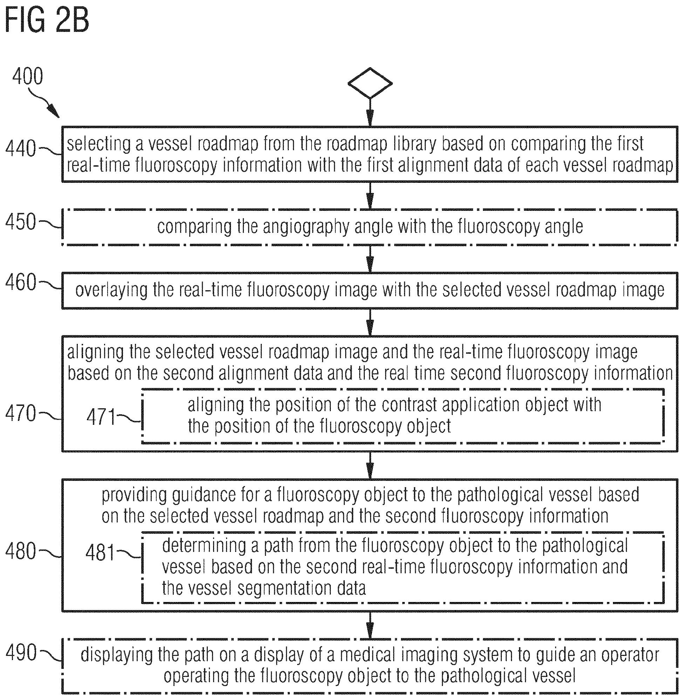

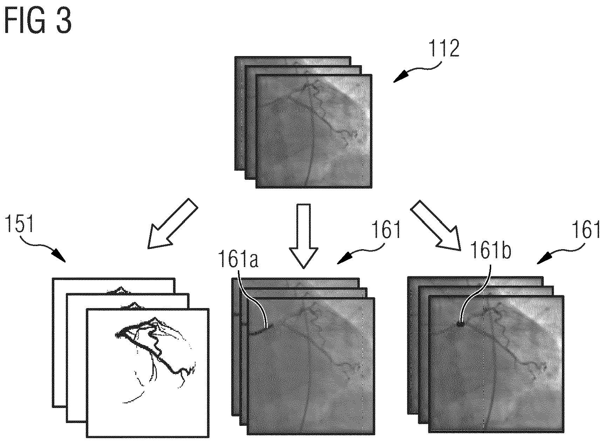

This general concept will be explained with reference to the appended drawings. Figs. 1, 1A and 1B illustrate the various aspects of the pathological vessel guidance workflow based on the pathological vessel guidance method while Figs. 2A and 2B illustrate the various step of the pathological vessel guidance method. Figs. 3 to 7 are used throughout the discussion of Figs. 1 as well as 2A and 2B to illustrate certain aspects of the workflow and the method, respectively.

Figs. 1, 1A and 1B shows a schematic diagram of a pathological vessel guidance workflow according to embodiments of the invention. Fig. 1 shows the orientation of Figs. 1A and 1B with regard to one another. The lines shown in Figs. 1A and 1B indicate where various data, such as vessel image 112 or the output of processing entities, such as vessel segmentation 150, is provided to. The blocks shown in Fig.1 indicate both processing entities, such as cardiac cycle detection 120, and stored data elements, such as vessel roadmap 211.

The workflow is separated into two phases, i.e., a roadmap generation phase 100 and a roadmap deployment phase 300. Roadmap generation phase 100 is shown in Fig. 1A. Roadmap deployment phase 300 is shown in Fig. 1B. During roadmap generation phase 100, a vessel image sequence 110 is processed to generate a vessel roadmap library 200. Vessel roadmap library 200 is then deployed in roadmap deployment phase 300 to provide a dynamic vessel roadmap for guidance of a fluoroscopy object during fluoroscopy. First, roadmap generation phase 100 will be described.

Vessel image sequence 110 may include a plurality of vessel images 112. Vessel images 112 are images of vessels or a vessel tree, such as the coronary arteries, the iliac veins or the lumbar lymph trunk. Generally, in the context of the present application, vessels may therefore refer to arteries, veins or lymphatic vessels. Vessel images 112 and more generally vessel image sequence 110 may be obtained using any medical imaging method capable of rendering vessels visible, such as angiography. In particular, medical imaging methods used to obtain vessel image sequence 110 may be based on injecting a radio-opaque contrast medium into vessels via a contrast application object and rendering the radio-opaque contrast medium visible via e.g., an x-ray. In some embodiments, vessel images 112 may be stored as or may be Digital Imaging and Communications in Medicine (DICOM) images.

Vessel image sequence 110 may include imaging physiological information 111. Imaging physiological information 111 may be any physiological information of a patient recorded while the imaging method used to obtain vessel images 112 is performed and which may be used to later overlay and align vessel roadmaps with a real-time fluoroscopy image. In particular, imaging physiological information 111 may be recorded at approximately the same point in time as the corresponding vessel image 112. In some embodiments, imaging physiological information 111 may include or may be an electrocardiogram (ECG) recorded while the imaging method used to obtain vessel images 112 is performed. In some embodiments, imaging physiological information may be stored as or may be a DICOM data tag included in the DICOM image file of the corresponding vessel image 112. Imaging physiological information 111 may be included in, e.g. stored as part of, first alignment data 212 of a vessel roadmap 210.

Vessel image sequence 110 may include imaging information 113. Imaging information 113 may indicate one or more parameters associated with an imaging method used to obtain vessel images 112. As discussed above, the imaging method used to obtain vessel images 112 may for example be angiography. Accordingly, imaging information 113 may include at least one of an angiography angle and a contrast medium dosage.

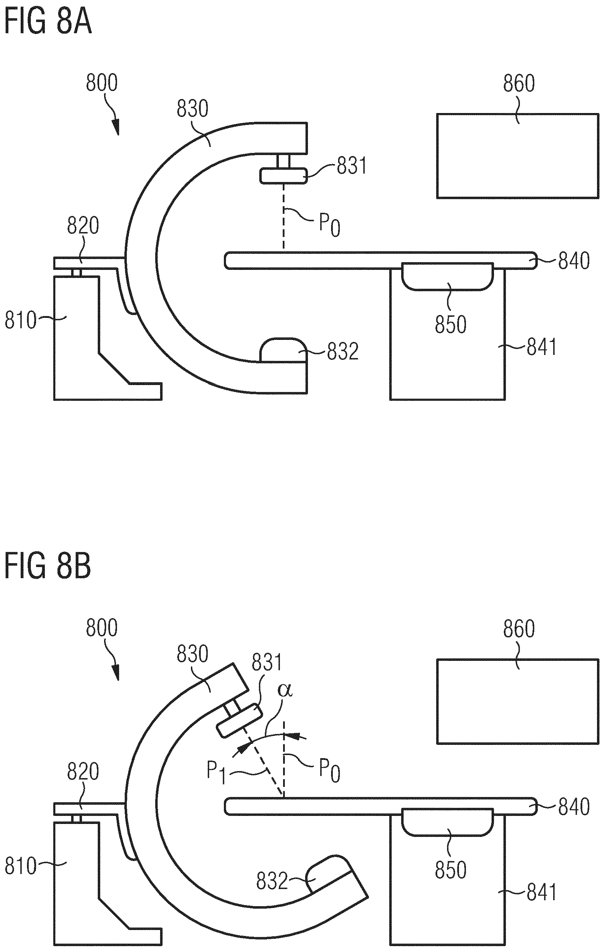

Briefly referring to Figs. 8A and 8B, both show a medical imaging system 800 including a rotatable C arm 830. X-ray emission means 831 and X-ray detection means 832 may be mounted on C arm 830. In Fig. 8A, C arm 830 is in a neutral position P₀, i.e. X-ray emission means 831 are located directly above a patient surface 840. In Fig. 8B, C arm 830 and thereby X-ray emission means 831 are rotated counterclockwise with respect to neutral position P₀ of C-arm 830 in Fig. 8A to a position P₁. The angle between position P₀ and position P₁, as indicated in Fig. 8B, is referred to as the angiography angle or the fluoroscopy angle, depending on the medical imaging process. It will be understood that the neutral position may be used as an imaging position. In such a case, the angiography angle is 0°. Further, in case of a single axis angiography system, the neutral position is typically defined as shown in Fig.8A. In multiple axis angiography systems, additional C arms may be present, such as a ceiling mounted C arm. In such a case, the neutral position may be defined as the position in which X-ray emission means 831 and X-ray detection means 832 are at the same level as a patient on patient surface 840.

While the above definition of the angiography angle is based on the position of X-ray emission means 831, the angiography angle may analogously be defined based on the position of X-ray detection means 832.

The contrast medium dosage may indicate the dosage of radio-opaque contrast medium administered to a patient in order to render the vessels of the patient visible during the imaging method. The contrast medium dosage may be measured in milliliters per kilogram of body weight. In the case of contrast media including iodine, the contrast medium dosage may also be measured in milligram iodine per kilogram of body weight.

Vessel images 112 may be processed by contrast detection 140 to detect contrasted vessel images among the plurality of vessel images 112. Contrast detection 140 may detect contrasted vessel images by detecting the presence of contrast medium in vessel images 112. Contrast detection 140 may in some embodiments analyze the pixels of each vessel image 112 to detect, based on a property of the pixels, the presence of contrast medium in the respective vessel image 112. The property of the pixels may e.g., a brightness value or a color value. In some embodiments, a vessel image 112 may be identified as a contrasted vessel image if a single pixel indicative of contrast medium is identified. In some embodiments, a vessel image 112 may be identified as a contrasted vessel image if a number of pixels indicative of contrast medium which exceeds a contrast medium detection threshold is identified. In some embodiments, contrast detection 140 may detect contrasted vessel images among the plurality of vessel images using a deep learning model, such as DeepNet, which identifies contrast medium in the vessel images 112 and provides a frame-by-frame classification of the vessel image sequence 110. In some embodiments, contrast detection 140 may also take into account the contrast medium dosage indicated by imaging parameters 114.

The contrasted vessel images detected by contrast detection 140 are provided to vessel segmentation 150 and contrast application segmentation 160. Furthermore, each contrasted vessel image may be included in, e.g., stored as part of, a vessel roadmap image 211 of vessel roadmap 210.

Vessel segmentation 150 may perform vessel segmentation on the contrasted vessel images to generate vessel segmentation data. Accordingly, vessel segmentation 150 may generate data indicative of the position and/or the course of the vessels within the contrasted vessel images. Vessel segmentation data may be generated by vessel generation 150 based on a variety of image segmentation approaches, such as based on convolutional neural networks (CNN), e.g. U-Net, densely connected neural networks, deep-learning methods, graph-partitioning methods, e.g. Markoff random fields (MRF), or region-growing methods, e.g. split-and-merge segmentation. The vessel segmentation data may then be included in, e.g. stored as part of, a vessel roadmap image 211 of a vessel roadmap 210.

It should be noted that, while vessel segmentation 150 is shown in Fig. 1 as processing contrasted vessel images, vessel segmentation may also directly process vessel images 112 to generate vessel segmentation data prior to detecting contrasted vessel images. In some embodiments, vessel segmentation 150 may also be incorporated into or be part of contrast detection 140. In such embodiments, detecting pixels indicative of contrast mediums may at the same time be used to generate vessel segmentation data.

Contrast application object segmentation 160 may perform contrast application object segmentation on the contrasted vessel images to generate contrast application object segmentation data. Accordingly, contrast application object segmentation data may identify a position and/or the course of the contrast application object in the contrasted vessel images. More precisely, the contrast application object segmentation data may in some embodiments be a catheter, which is used to apply the contrast medium. In such embodiments, the contrast application object segmentation data may indicate the position and/or the course of the tip of the catheter, the body of the catheter or the guidewire of the catheter. Contrast application object segmentation data may, like vessel segmentation data, be generated based on a variety of image segmentation approaches, including, but not limited to, U-Net or MRF. The contrast application object segmentation data may then be included in, e.g. stored as part of, second alignment data 213 of vessel roadmap 210.

While contrast application object segmentation 160 is shown in Fig. 1 as processing contrasted vessel images, contrast application object segmentation 160 may also directly process vessel images 112.

Both vessel segmentation 150 and contrast application object segmentation 160 perform image segmentation on vessel images 112 or contrasted vessel images, respectively. To better illustrate possible segmentations performed on vessel images 112 or contrasted vessel images, respectively, Fig. 3 shows three example segmentations of a vessel image 112. From left to right, Fig. 3 shows an example of vessel segmentation data 151 and two examples of contrast application object segmentation data 161. As can be seen, vessel segmentation data 151 indicate the position and the course of the vessels in vessel image 112. The left vessel segmentation data 161 indicate the position and the course of a catheter body 161a. The right vessel segmentation data 161 indicate the position of a catheter tip 161b.

Pathological vessel detection 170 detects pathological vessels in the contrasted vessel images. To this end, pathological vessel detection 170 performs image segmentation on the contrasted vessel images to identify areas within the contrasted vessel images, which include pathological vessel segments. In one example, pathological vessel detection 170 may detect one or more pathological vessels based on any one of the image segmentation approaches trained on vessel images with annotated pathological vessels. The pathological vessels detected by pathological vessel detection 170 may then be included as pathological vessel data 215 in vessel roadmap 210.

Pathological conditions in vessels often affect the lumina of the vessel. Accordingly, pathological vessel detection 170 may in a further example detect pathological vessels based on their lumina. Accordingly, pathological vessel detection 170 may first determine centerlines of the vessels included in the contrasted vessel images. Then, pathological vessel detection 170 may determine, based on the centerlines, lumina of the vessels included in each vessel roadmap image of the vessel roadmap library. Finally, pathological vessel detection 170 may detect one or more pathological vessels based on the lumina of the vessels included in the contrasted vessel images.

A pathological vessel in the context of the present application may be any vessel experiencing a pathological condition, such as a stenosis, a lesion, a vasoconstriction or a vasodilation.

Pathological vessel detection 170 may further determine, e.g., based on the determined lumen of the pathological vessel, the grade of a pathological condition, e.g. a stenosis in a pathological vessel, as e.g., defined by the Society of Cardiovascular Computed Tomography (SCCT). Accordingly, pathological vessel detection 170 may, based on a comparison of the total lumen with the unobstructed lumen, determine the grade of the stenosis. It will be understood that pathological vessel detection 170 may be able to also grade stenoses in vessels other than the coronary arteries based on the grading system applicable to such vessels.

In some cases, pathological vessel detection 170 may detect more than one pathological vessel. In such cases, pathological vessel detection 170 may further be able to determine in which order the pathological vessels may be treated during a medical intervention. Such a determination may for example be based on a starting position of a medical intervention and the position of the pathological vessel relative to the starting position or may be based on the grading of the severity of the pathological conditions of the pathological vessels discussed above.

It will be understood that pathological vessel detection 170 may also perform the detection based on vessel images 112 directly or may be integrated with any one of the other processing entities of roadmap generation phase 100 performing image segmentation, i.e., contrast detection 140, vessel segmentation 150 and contrast application object segmentation 160.

Imaging physiological information 111 may be processed by cardiac cycle detection 120. Cardiac cycle detection 120 may identify one or more cardiac cycles within imaging physiological information 111. Cardiac cycle detection 120 may identify at least one cardiac cycle within imaging physiological information 111 by detecting a first R peak and a second R peak within imaging physiological information 111. The first and the second R peak may indicate the start and the end, respectively, of a cardiac cycle. It should be understood that any other graphical deflection of an ECG may be used to indicate the start and the end of a cardiac cycle. Accordingly, cardiac cycle detection may e.g. detect a first and a second P wave.

In some embodiments, detecting the first R peak and the second R peak is based on a combined adaptive electrical activity threshold, which may be the sum of a steep-slope threshold, an integration threshold and an expected beat threshold. In such embodiments, an R peak may be detected if the amplitude of a complex lead is equal to or above an amplitude value of a complex lead, e.g. derived from a 12-lead. Of course, any type of ECG lead may be used to obtain an amplitude of a complex lead, such as a Wilson or a Goldberger ECG.

The steep-slope threshold may initially be set at 60% of the maximum of the amplitude value of the complex lead measured during an initialization period, e.g. 5 s. Once the steep slope threshold as well as the other two thresholds have been initialized, a first QRS complex is detected. During some recalculation interval after detection of the first QRS complex, e.g. 200 ms, the steep-slope threshold may be set at 60% of the maximum of the amplitude value of the complex lead measured during the recalculation interval. Afterwards, the steep-slope threshold may be decreased during a decrease interval, e.g. 1000 ms, i.e. 1200 ms after detection of the first QRS complex, to 60% of its value, i.e. 36% of the measured amplitude of the complex lead. Afterwards, the steep-slope threshold remains at 36%. The decrease may be stopped once a new QRS complex is detected, assuming that the decrease interval has not expired, and the steep-slope threshold may be recalculated as described above. In some embodiments, the steep slope threshold may be set to an average of the currently calculated 60% of the maximum of the amplitude value of the complex lead measured during the recalculation interval and the preceding four calculated values. In some embodiments, the recalculated value of the steep-slope threshold may further be limited to 110% of the previous value of the steep-slope threshold if the recalculated value exceeds 150% of the previous value.

The integration threshold may initially be set to the mean value of the first derivative of the amplitude of the complex lead with regard to time during an integration threshold initialization interval, which may e.g., be 350 ms. Afterwards, the integration threshold is updated based on an update time interval, which may be 350 ms. For every update time interval, the integration threshold may be calculated as the sum of the previous integration threshold and an update value. The update value may be calculated by subtracting from the maximum value of the amplitude of the complex lead during an initial period of the update time interval, e.g., 50 ms, the maximum value of the amplitude of the complex lead during a final period of the update time interval, e.g., 50 ms. The update value may further be divided by a weighting factor, which may e.g., be 150.

The expected beat threshold may initially be set to zero. To determine the expected beat threshold, the average time difference between R peaks may be calculated. In some embodiments, the average time difference may be based on the previous five time differences between R peaks. After a fraction, such as two-thirds, of the average time difference sine the last R peak has expired, the expected beat threshold may be reduced at a fraction of the rate at which the steep-slope threshold may be decreased during the decrease time interval, e.g. at 70% percent of the decrease of the steep-slope threshold. Once the average time difference has expired, the expected beast threshold remains constant. Upon detection of a new R peak, the expected beat threshold may be re-set to zero and be decreased during the next cardiac cycle based on a re-calculated average time difference between R peaks as discussed above. Accordingly, the expected beat threshold may decrease the combined adaptive electrical activity threshold toward the expected end of a cardiac cycle.

It should be noted that the above discussion of how a cardiac cycle may be detected based on combined adaptive electrical activity threshold is merely provided as an example. Cardiac cycle detection 120 may detect cardiac cycles using any suitable analysis of an ECG. For example, cardiac cycle detection 129 may detect cardiac cycles based on performing different types of analyses based transforms, such as short-time Fourier-transform or based on deriving event vectors from the ECG and providing decision rules, which determine cardiac cycles based on the derived event vectors.

The one or more cardiac cycles detected by cardiac cycle detection 120 may be included in, e.g. stored as part of, first alignment data 212. In addition, the one or more cardiac cycles detected by cardiac cycle detection 120 may be provided to EDR detection 130.

EDR detection 130 may derive, based on the one or more cardiac cycles, an electrocardiogram derived respiratory (EDR) signal. The EDR signal may be derived by observing fluctuations between detected different cardiac cycles. Generally speaking, inhaling typically increases and exhaling typically decreases the heart rate. More precisely, such fluctuations can in some embodiments be used to derive the EDR signal by computing for the one or more detected cardiac cycles, the R-to-S peak. The R-to-S peak corresponds to the amplitude of the EDR signal. The R-to-S-peaks are then interpolated using cubic splines to obtain the EDR signal. It should be understood that in some embodiments, other approaches may be used to obtain an EDR signal from the imaging physiological information 111. The EDR signal may be included in, e.g. stored as part of, second alignment data 213.

It should be noted that in some embodiments, EDR detection 130 may be omitted. In such embodiments, second alignment data 213 may only include contrast application object segmentation data. Further, in some embodiments EDR detection 130 may be included in cardiac cycle detection 120. For example, the approach used by cardiac cycle detection 120 to identify one or more cardiac cycles may also be able to directly provide an EDR signal or be able to provide an EDR signal based on an intermediate step of the approach.

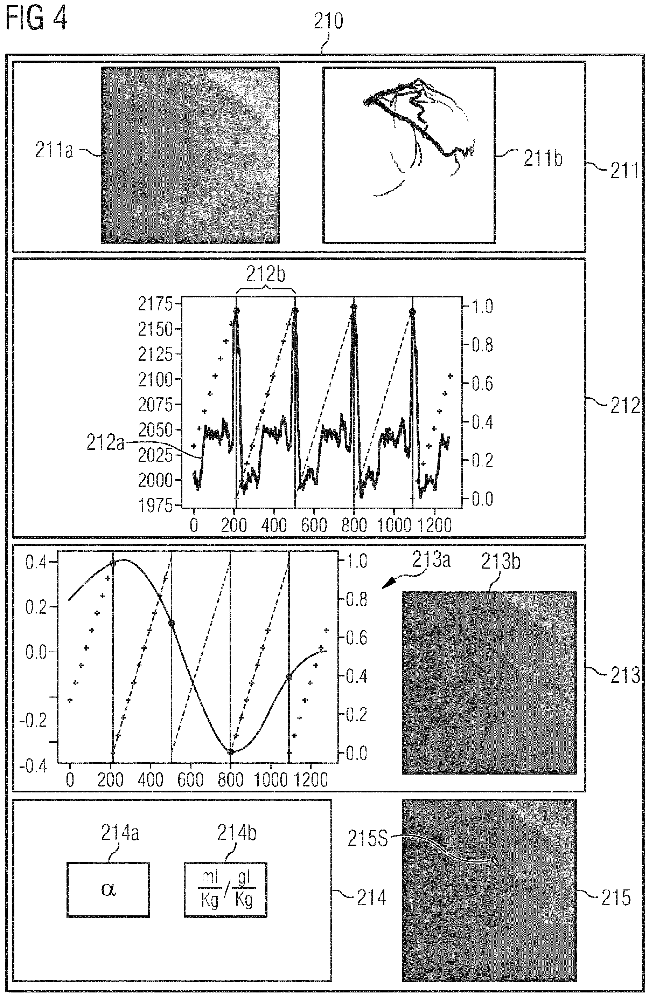

As mentioned above, the output of processing entities 120 to 170 forms part of vessel roadmaps 210, which are part of vessel roadmap library 200. To illustrate the data, which may be included in a vessel roadmap 210, Fig. 4 shows an example vessel roadmap 210, including a vessel roadmap image 211, first alignment data 212, second alignment data 213, imaging parameters 214 and pathological vessel information 215.

Vessel roadmap image 211 may include a vessel image 211a. Vessel roadmap image 211a corresponds to a vessel image 112 of the vessel image sequence 110, which has been identified by contrast detection 140 as including contrast. Further, vessel roadmap image 211 may include vessel segmentation data 211b generated by vessel segmentation 150. Both vessel roadmap 211a and vessel segmentation data 211b may be used as the roadmap to be laid over a fluoroscopy image during the roadmap deployment phase 300 by vessel overlay roadmap 381 discussed later. Accordingly, vessel roadmap image 211 is the part of vessel roadmap 211 providing the actual vessel roadmap. In some embodiments, vessel roadmap image 211 may correspond to a vessel image 112 recorded as part of the vessel image sequence 110 as well as an image indicating the vessel segmentation data generated by vessel segmentation 150. In some embodiments, vessel roadmap image 211 may only include an image indicating the vessel segmentation data generated by vessel segmentation 150, as illustrated by vessel segmentation data 211b in Fig. 4. In some embodiments, vessel roadmap image 211 may be data indicating the position of vessel pixels, which may subsequently be used to highlight corresponding pixels in a fluoroscopy image as vessel pixels.

First alignment data 212 may include imaging physiological information 111 as recorded as part of the vessel image sequence 110. Since imaging physiological information 111 may in some embodiments be an ECG, imaging physiological information 111 is shown in Fig. 4 as an alignment ECG 212a. Further, first alignment data 212 may include one or more cardiac cycles as detected by cardiac cycle detection 140. In Fig. 4, a cardiac cycle 212b is illustrated as indicted by adjacent R peaks in alignment ECG 212a. While first alignment data 212 of vessel roadmap 210 is shown here as based on an ECG curve, it should be understood that alignment ECG 212a and cardiac cycle 212b may typically be stored within second alignment data 212 as an indication of the ECG value, i.e., e.g. the amplitude of the complex lead discussed above, and the position of the ECG value within a cardiac cycle.

Accordingly, the first alignment data may in some embodiments not include the entire alignment ECG 212a. Instead, first alignment data 212 may e.g., be a tuple with the first value indicating the amplitude and the second value indicating the position within a cardiac cycle. In some embodiments, if more than one cardiac cycle has been identified. Fist alignment data 212 may be a triple with the third value identifying the respective cardiac cycle. In some embodiments, in which cardiac cycle detection 140 is omitted, the cardiac cycle information may be replaced by temporal information indicating an associated point in time of the ECG value relative to other vessel roadmaps 210.

More generally, first alignment data 212 provide data to enable aligning vessel roadmap image 211 with a fluoroscopy image. Vessels shift for a variety of factors, including, but not limited to, cardiac muscle contraction and relaxation, i.e., cardiac motion, as well as breathing. First alignment data 212 enable compensation of vessel shift caused by cardiac motion. To this end, first alignment data 212 provide physiological information relating to the heart. It should therefore be understood that first alignment data 212 generally provide data enabling an alignment of a vessel roadmap with a fluoroscopy image necessitated due to vessel shifts caused by cardiac motion. First alignment data 212 may thus include any cardiac information necessary to compensate such vessel shifts.

Second alignment data 213 may include an EDR signal 213a generated by EDR detection 130, and contrast application object segmentation data 213b generated by contrast application object segmentation 160. EDR signal 213a is shown as a curve in Fig. 4 in order to illustrate EDR signal 213a. However, similarly to the discussion of alignment ECG 212a, EDR signal 213a may in some embodiments be a data set indicating the value of EDR signal 213a as well as the temporal position of the value within the EDR signal curve. Further, as shown in Fig. 4, contrast application object segmentation data 213b may in some embodiments be an image indicating the vessel segmentation data. In some embodiments, contrast application object segmentation data 213b may be data indicating the position of contrast application object pixels.

More generally, second alignment data 213 provide data to enable aligning vessel roadmap image 211 with a fluoroscopy image. While first alignment data 212 are described above as compensating vessel shift caused by cardiac motion, second alignment data 213 may compensate for vessel shift caused by breathing motion. To this end, second alignment data 213 provide both physiological information relating to the breathing and information relating to the position of the contrast application object, which may be shifted due to breathing. It should therefore be understood that second alignment data 213 generally provide data enabling an alignment of a vessel roadmap with a fluoroscopy image necessitated due to vessel shifts caused by breathing motion. Second alignment data 213 may thus include any breathing-related information necessary to compensate such vessel shifts. For example, in some embodiments, second alignment data 213 may include only one of EDR signal 213a and contrast application object segmentation data 213b since in some embodiments only one of the two may be sufficient to compensate vessel shifts cause by breathing motion. For example, in some embodiments EDR signal 213a may be omitted.

As discussed above, contrast application object segmentation data 213b is generated by contrast application object segmentation 160. Accordingly, second alignment data 213 is in some embodiments at least derived from vessel roadmap image 211. In embodiments, in which EDR signal 213a is also present, second alignment data 213 may further be derived from first alignment data 212 in addition to being derived from vessel image 211.

Imaging parameters 214 may include imaging parameters 113 associated with the imaging method used to obtain vessel image 112 included as the vessel roadmap image 211. As shown in Fig. 4, imaging parameters 214 may thus include angiography angle 214a and contrast medium dosage 214b.

Pathological vessel information 215 is generated by pathological vessel detection 170. Accordingly, pathological vessel information 215 indicates pathological vessels in vessel roadmap 212. As illustrated in Fig. 4, pathological vessel information 215 may e.g. correspond to vessel roadmap 212 with a highlighted region 215S indicating a pathological vessel. In addition to or instead of, pathological vessel information 215 may in some embodiments include data identifying pixels in vessel roadmap 212, which are part of a pathological vessel segment. Generally speaking, pathological vessel information 215 may be any kind of information indicating which of the vessel visible in vessel roadmap 212 is a pathological vessel.

Vessel roadmap library 200 is the output generated by roadmap generation phase 100. This output may subsequently be deployed during roadmap deployment phase 300. In some embodiments, roadmap deployment phase 300 may be a medical intervention, such as a percutaneous coronary intervention (PCI). PCI is performed using fluoroscopy. Accordingly, vessel roadmap library 200 can be laid over the real-time fluoroscopy images during the PCI in order to guide a medical specialist through the coronary arteries to e.g., a stenosis without having to use a contrast medium. In such embodiments, roadmap generation phase 100 may include coronary angiography, which is used to obtain a coronary angiogram. The coronary angiogram in such embodiments corresponds to vessel image sequence 110. Since roadmap deployment phase 300 may be a medical intervention, it may also be referred to as an online phase, while roadmap generation phase 100 may also be referred to as an offline phase.

Roadmap deployment phase 300 performs real-time fluoroscopy 310 in order to obtain a real-time fluoroscopy image 311 and corresponding real-time first fluoroscopy information 312, real-time second fluoroscopy information 313 and imaging parameters 314.

Real-time fluoroscopy image 311 may be an image obtained using real-time fluoroscopy 310. During real-time fluoroscopy 310, no contrast medium needs to be injected into vessel or a vessel tree given that vessel images are provided by vessel roadmap library 200. Accordingly, since fluoroscopy is typically performed using X-ray, the only radio-opaque structure visible in the real-time fluoroscopy image 311, apart from e.g. bones of the patient, is a fluoroscopy object. Like vessel image 112, real-time fluoroscopy image 311 may be stored as a DICOM image.

First fluoroscopy information 312 may be any kind of fluoroscopy physiological information of a patient on whom real-time fluoroscopy 310 is performed and which may be used to overlay and align vessel roadmaps 211 with fluoroscopy image 311. In some embodiments, first fluoroscopy information 312 may include an ECG recorded while real-time fluoroscopy 310 is performed. In such embodiments, first fluoroscopy information 312 may be processed by cardiac cycle detection 320 to identify one or more cardiac cycles based on the ECG. The identified one or more cardiac cycles may then also be included in the real-time fluoroscopy information 312. Cardiac cycle detection 320 may detect one or more cardiac cycles in the fluoroscopy ECG in the same manner as cardiac cycle detection 140 may detect one or more cardiac cycles in the ECG recorded while vessel image sequence 110 is obtained.

The one or more cardiac cycles identified by cardiac cycle detection 320 may be processed by EDR detection 330. EDR detection 330 may derive an EDR signal based on the identified one or more cardiac cycles in the same manner as describe with respect to EDR detection 130. The derived EDR signal may then be included in second real-time fluoroscopy information 313.

As discussed above, in some embodiments EDR detection 130 may be omitted. In such embodiments, EDR detection 330 may additionally derive an EDR signal based on the one or more cardiac cycles included in first alignment data 212 of a vessel roadmap 210 selected by roadmap selection 371, which will be discussed in more detail below. It should be noted that EDR detection 330 may also be omitted if EDR detection 130 is omitted. In such embodiments, vessel roadmap selection 370 and vessel roadmap alignment 282 may operate without any EDR signals.

In addition to the EDR signal, second real-time fluoroscopy information 313 may include fluoroscopy object segmentation data identifying a position of a fluoroscopy object in fluoroscopy image 311. The fluoroscopy object may be a fluoroscopy catheter, which may e.g., be used during a medical intervention, such as PCI. As opposed to the contrast application object, which may also be a catheter, the fluoroscopy catheter may typically not be used to inject contrast medium into a vessel, though it may still be configured for that purpose. The fluoroscopy segmentation data may be generated by fluoroscopy segmentation 360 based on a variety of image segmentation approaches as discussed above with regard to vessel segmentation 150 and contrast application object 160, such as based on CNNs or MRFs.

Imaging parameters 314 may indicate one or more parameters associated with an imaging method used to obtain real-time fluoroscopy image 311. Accordingly, imaging parameters 314 may correspond to imaging parameters 113 and may thus include at least one of a fluoroscopy angle and a contrast medium dosage.

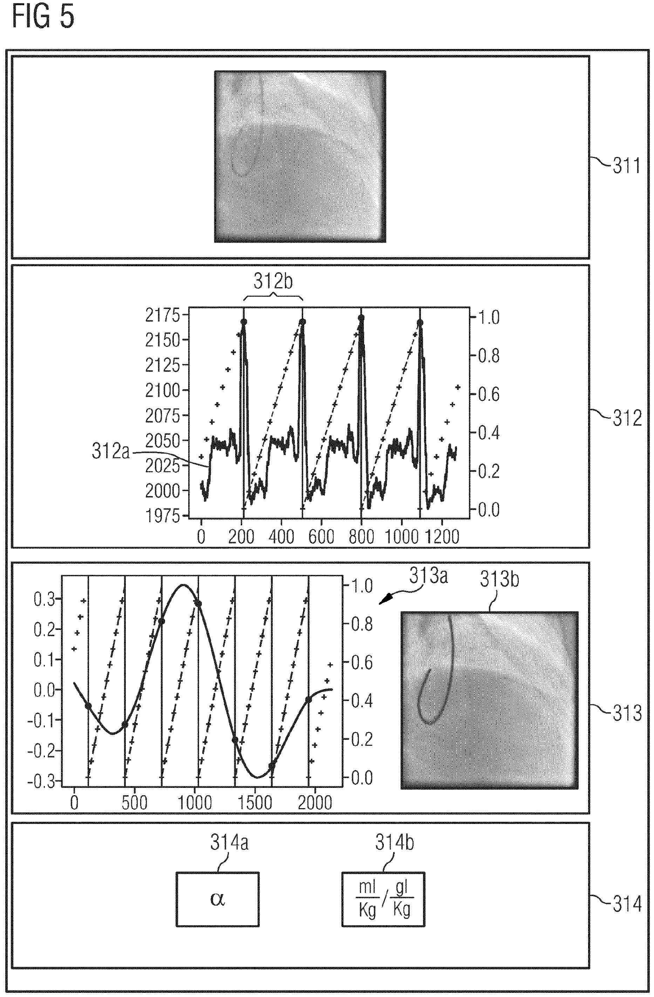

The data recorded by real-time fluoroscopy 310 and processed by processing entities 320, 330 and 360 is illustrated in Fig. 5.

As shown in Fig. 5, real-time fluoroscopy image 311 includes the fluoroscopy image recorded by real time fluoroscopy 310. Typically, only the fluoroscopy object is visible in real-time fluoroscopy image 311, unless other radio-opaque structures of the patient are visible.

First fluoroscopy information 312 includes fluoroscopy ECG 312a recorded during real-time fluoroscopy and one or more cardiac cycles 312b identified by cardiac cycle detection 320. Analogously to first alignment data 212, first fluoroscopy information 312 may include any cardiac information necessary to compensate vessel shifts when laying one of the vessel roadmap images 211 over real-time fluoroscopy image 311. Accordingly, in some embodiments, first fluoroscopy information 312 may only include fluoroscopy ECG 312a or may include other or additional information to compensate vessel shifts when laying one of the vessel roadmap images 211 over real-time fluoroscopy image 311. Further, while fluoroscopy ECG 312a is illustrated as the ECG curve recorded during real-time fluoroscopy 310, in some embodiments only values corresponding to the point on the ECG curve may be included in first real-time fluoroscopy information 312, e.g. a tuple or a triple, respectively, including the amplitude value of the complex lead of the fluoroscopy ECG 312a, the temporal position within the ECG and an indication of the identified cardiac cycle.

Second real-time fluoroscopy information 313 may include a fluoroscopy EDR signal 313a generated by EDR detection 330 and contrast application object segmentation data 313b generated by contrast application object segmentation 360. EDR signal 313a is shown as a curve in Fig. 5 in order to illustrate fluoroscopy EDR signal 313a. However, similarly to the discussion of preceding discussions of curves, i.e. alignment ECG 212a, EDR signal 213a and fluoroscopy ECG 312a, fluoroscopy EDR signal 313a may in some embodiments be a data set indicating the value of fluoroscopy EDR signal 313a as well as the temporal position of the value within the fluoroscopy EDR signal curve. Further, as shown in Fig. 5, fluoroscopy object segmentation data 313b may in some embodiments be an image indicating the vessel segmentation data. In some embodiments, fluoroscopy object segmentation data 313b may be data indicating the position of fluoroscopy object pixels.

The data obtained by real-time fluoroscopy 310 and vessel roadmap library 200 may be provided to a vessel roadmap selection 370. Vessel roadmap selection 370 may select one of the vessel roadmaps 210 from roadmap library 200 based on comparing first real-time fluoroscopy information 312 with first alignment data 212 of each vessel roadmap 210 in roadmap library 200.

More precisely, vessel roadmap selection 370 may compare alignment ECG 212a of each vessel roadmap 210 with fluoroscopy ECG 312a to determine a vessel roadmap 210 approximately corresponding, in terms of the respective alignment ECG 212a, to fluoroscopy ECG 312a. By selecting a vessel roadmap based on an ECG comparison, a vessel roadmap 210 can be chosen in which the position of the vessels is most similar, due to a similar shift caused by similar cardiac motion, to the position of the vessels in real time fluoroscopy image 311.

In addition, in some embodiments vessel roadmap selection 370 may select a vessel roadmap 210 from roadmap library 200 based on comparing second real-time fluoroscopy information 313 with the second alignment data 213 of each vessel roadmap 210. More precisely, vessel roadmap selection 370 may additionally compare EDR signal 213a of each vessel roadmap 210 with fluoroscopy EDR signal 313a to determine a vessel roadmap 210 approximately corresponding, in terms of the respective EDR signal 213a, to fluoroscopy EDR signal 313a. By selecting a vessel roadmap additionally based on an EDR signal comparison, a vessel roadmap 210 can be chosen in which the position of the vessels is most similar, due to a similar shift caused by similar breathing motion, to the position of the vessels in real time fluoroscopy image 311.

In summary, vessel roadmap selection 370 may, based on an ECG comparison and in some embodiments based on an additional EDR comparison, select a vessel roadmap 210 from vessel roadmap library 200. The vessels visible in the accordingly selected vessel roadmap 210 have experienced a similar shift due to cardiac motion as well as due to breathing motion. The position of the vessels visible in the accordingly selected vessel roadmap 210 may thus be approximately similar to the position of the vessels in real-time fluoroscopy image 311.

Angle comparison 380 may compare the angiography angle included in the imaging parameters 214 of vessel roadmap 210 with the fluoroscopy angle included in imaging parameters 314 of real-time fluoroscopy 310. The comparison performed by angle comparison 380 may ensure that the view provided by selected vessel roadmap image 211 and the view provided by real-time fluoroscopy image 311 are obtained at approximately the same C-arm position. If the angiography angle and the fluoroscopy angle are approximately the same, the views provided by selected vessel roadmap image 211 and real-time fluoroscopy image 311 are approximately similar. If the angiography angle and the fluoroscopy angle differ, e.g., differ by more than an angle difference threshold, the views provided by selected vessel roadmap image 211 and real-time fluoroscopy image 311 may be too different. Accordingly, in such cases angle comparison 380 may decide to refrain from overlaying vessel roadmap image 211 over real-time fluoroscopy image 311. Due the deviation of the views in 3D corresponding to the difference of the angles, vessel roadmap overlay 381 and vessel roadmap alignment 382 may not be able properly overlay and align the views. The angle difference threshold may e.g., be 5° or 2°.

Angle comparison 380 may in some embodiments indicate, via a display, such as display 350, to a medical specialist operating a medical imaging device, such as medical imaging device 800, that the angiography angle and the fluoroscopy angle differ. Angle comparison 380 may further indicate how to correct the fluoroscopy angle by indicating the proper angular position to which C arm 830 of medical imaging device 300 should be moved. In some embodiments, angle comparison 380 may also be configured to control C arm 830 and may thus be configured to move C arm 830 to the proper angular position.

Vessel roadmap overlay 381 may lay the selected vessel roadmap image 211 of the selected vessel roadmap 210 over the real-time fluoroscopy image 311. In some embodiments, vessel roadmap overlay 381 may perform the overlay by superimposing vessel image 211a over real-time fluoroscopy image 311. Superimposing vessel image 211a may in some embodiments be achieved by transparent color blending, i.e. two pixel values from vessel image 211a, one corresponding to the pixel value as recorded originally in corresponding vessel image 112 and one corresponding to a color selected for vessel representation, can be simultaneously shown. In some embodiments, vessel roadmap image 211a may be overlaid with a variable level of opacity. In some embodiments, vessel segmentation data 211b may be integrated into real-time fluoroscopy image 311, e.g. by changing the values of the pixels indicated as vessel pixels by vessel segmentation data 211b.

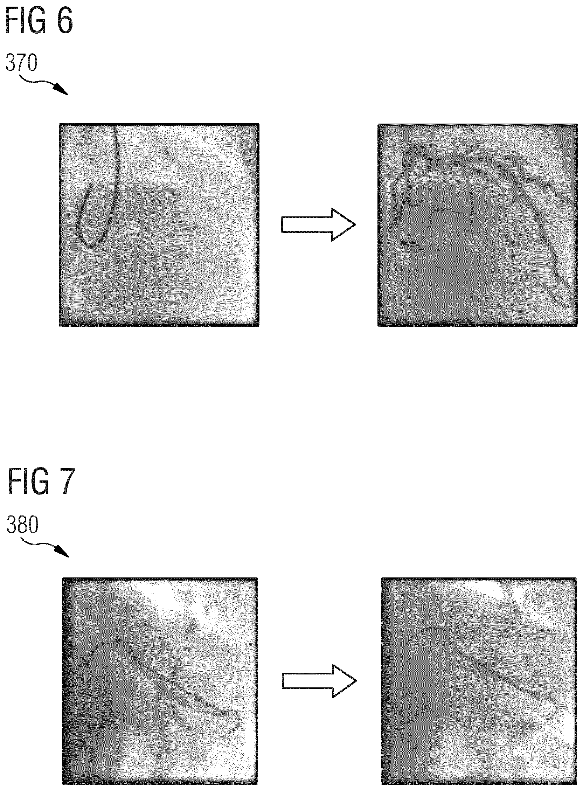

Fig. 7 provides an example of vessel roadmap overlay 381. On the left, real-time fluoroscopy image 311 with a visible fluoroscopy object can be seen prior the overlay of a vessel roadmap image 211. On the right, real-time fluoroscopy image 311 can be seen after the overlay of a vessel roadmap image 211.

Roadmap image 211 laid over real-time fluoroscopy image 311 by vessel roadmap overlay 381 may be aligned by vessel roadmap alignment 382. Vessel roadmap alignment 382 aligns overlaid vessel roadmap image 211 and real-time fluoroscopy image 311 based on second alignment data 213 and real time second fluoroscopy information 313. In particular, vessel roadmap alignment 382 aligns the position of the contrast application object with the position of the fluoroscopy object based on contrast application object segmentation data 213b and fluoroscopy object segmentation data 313b. In other words, vessel roadmap alignment 382 aligns the positions of the contrast application object and the fluoroscopy object, which may both be catheters. For example, both object segmentation data 213b and fluoroscopy object segmentation data 313b may each indicate, in some embodiments, a centerline of the respective catheter. In such embodiments, vessel roadmap alignment 380 aligns vessel roadmap 211 with real-time fluoroscopy image 311 by minimizing the sum of squared distances between closest points on the centerlines of the catheters. It should be noted that aligning may include in-plane rotation, i.e. rotating the roadmap to achieve better alignment. By aligning the positions of the contrast application object with the position of the fluoroscopy object, any vessel shift caused by breathing motion may be further compensated in order to further improve the accuracy of overlaid vessel roadmap 210.

Fig. 7 provides an example of an alignment by vessel roadmap alignment 382 based on catheter centerlines. As can be seen on the left side of Fig. 7, prior to alignment by vessel roadmap alignment 382, the centerlines are apart from one another. After alignment by vessel roadmap alignment 382, the centerlines are approximately in the same position.

Finally, pathological vessel guidance 383 providing guidance for the fluoroscopy object to the pathological vessel detected by pathological vessel detection 170 based on the selected vessel roadmap 211 and second fluoroscopy information 313. To this end, pathological vessel guidance 383 may determine a path from the fluoroscopy object to the pathological vessel based on the second real-time fluoroscopy information 313, which includes the fluoroscopy segmentation data, and the vessel segmentation data 211b included in the selected vessel roadmap image 211. In other words, the path may indicate the vessel segments the fluoroscopy object needs to pass through in order to reach the pathological vessel from the current position of the fluoroscopy object within the vessels of a patient. Accordingly, pathological vessel guidance 383 may provide guidance for the fluoroscopy object by indicating the vessel segments through which the fluoroscopy object needs to pass in order to reach the pathological vessel.

Pathological vessel guidance 383 may in some embodiments. provide the guidance to a medical specialist operating the fluoroscopy object e.g., by displaying the path determined by pathological vessel guidance 383 on a display, such as display 860, to the medical specialist.

In summary, vessel roadmap selection 370, vessel roadmap overlay 381 and vessel roadmap alignment 382 select, overlay and align one of the vessel roadmaps 210 with fluoroscopy image 311 in order to provide a vessel roadmap during roadmap deployment phase 300. By taking into account first alignment data 212, second alignment data 213, the first fluoroscopy information 312 and the second fluoroscopy information 313, a vessel roadmap can be selected, overlaid and aligned with fluoroscopy image 311, which corresponds to the actual position of the vessels in fluoroscopy image 311 without having to inject contrast medium. Thus, vessel roadmap selection 370, vessel roadmap overlay 381 and vessel roadmap alignment 382 compensate any motion of the vessels, such as cardiac motion or breathing motion, in order to correctly overlay one of the vessel roadmaps 210 over fluoroscopy image 311. Further, the duration of the fluoroscopy may be reduced based on generated vessel roadmap library 200, thereby reducing radiation exposure due to the guidance provided by pathological vessel guidance 383, which provides direct guidance from the position of the fluoroscopy objection to the pathological vessel. This guidance reduces the durations of medical interventions, such as PCI, since guidance based on the properly selected, overlaid and aligned roadmap may enable fast and reliable navigation with the fluoroscopy object through the vessels.

Figs. 2A and 2B illustrate a pathological vessel guidance method 400 implemented by the pathological vessel guidance workflow 100 of Fig. 1. Steps shown as boxes inside boxes of other steps indicate sub-steps. Steps illustrated as dashed boxes in Figs. 2A and 2B indicate optional steps of method 400.To avoid repetitions, the discussion of Figs. 2A and 2B will only outline additional details if not already included in the discussion of the workflow 100 and will otherwise focus on identifying which processing entities of workflow 100 of Fig. 1 performs which steps of method 400.

In step 410, method 400 generates a vessel roadmap library. Step 410 is performed by processing entities 120 to 170 implementing roadmap generation phase 100 as discussed above.

In particular, step 410 may include a step 411, in which method 400 may record, for each vessel roadmap image, one or more imaging parameters 113.

Step 410 may further include a step 412, in which method 400 may obtain a vessel image sequence 110 and associated imaging physiological information 111.

Step 410 may further include a step 413, in which method 400 detects, within vessel image sequence 110, contrasted vessel images. Step 413 may be performed by contrast detection 140.

Step 410 may further include a step 414, in which method 400 may perform vessel segmentation on the contrasted vessel images to generate vessel segmentation data. Step 414 may be performed by vessel segmentation 150.

Step 410 may further include a step 415, in which method 400 may perform contrast application object segmentation on the contrasted vessel images to generate contrast application object segmentation data. Step 415 may be performed by contrast application object segmentation 160.

Step 410 may include a step 416, in which method 400 may generate a vessel roadmap 210 for each contrasted vessel image. More precisely, method 400 may in step 416 generate a vessel roadmap 210 by including the contrasted vessel image and the vessel segmentation data in vessel roadmap image 211, the imaging physiological information in first alignment data 212 and the contrast application object segmentation data in second alignment data 213.

Step 410 may finally include a step 417, in which method 400 may identify one or more cardiac cycles within the vessel image sequence based on an ECG. Step 417 may be implemented by cardiac cycle detection 120.

In step 420, method 400 detects, within the vessel roadmap images of the vessel roadmap library, a pathological vessel. Step 420 is performed by pathological vessel detection 170. Step 420 may include sub-steps 421, 422 and 423. In step 421, method 400 may determine centerlines of the vessels included in each vessel roadmap image. In step 422, method 400 may determine, based on the centerlines, lumina of the vessels included in each vessel roadmap image. In step 423 method 400 may detect the pathological vessel based on the lumina of the vessels included in each vessel roadmap image. Sub-steps 421 to 423 may likewise be performed by pathological vessel detection 170.

In step 430, method 400 obtains real-time fluoroscopy image 311 and corresponding real-time first fluoroscopy information 312 and real-time second fluoroscopy information 313. Step 430 may be performed by roadmap deployment phase 300 using medical imaging device 800 of Figs. 8A and 8B. Step 430 may include sub-steps 431 to 433. In step 431, method 400 may obtain fluoroscopy physiological information associated with real-time fluoroscopy image 311. The fluoroscopy physiological information may be included in first real-time fluoroscopy information 313. Step 431 may, like step 430, be performed by roadmap deployment phase 300 using medical imaging device 800 of Figs. 8A and 8B. In step 432, method 400 may perform fluoroscopy object segmentation on the fluoroscopy image to generate fluoroscopy object segmentation data. Step 432 may be performed by fluoroscopy object segmentation 360. In step 433, method 400 may identify one or more cardiac cycles based on the ECG in embodiments, in which first fluoroscopy information 312 includes an ECG. Step 433 may be performed by cardiac cycle detection 320.

In step 440, method 400 selects a vessel roadmap from the roadmap library based on comparing first real-time fluoroscopy information 312 with first alignment data 212 of each vessel roadmap 200. Step 440 is performed by vessel roadmap selection 370.

In step 450, method 400 may compare the angiography angle with the fluoroscopy angle and may refrain from overlaying real-time fluoroscopy image 311 with selected vessel roadmap image 211 if the angiography angle and the fluoroscopy angle differ by more than an angle difference threshold. Step 450 may be performed by angle comparison 380.

In step 460, method 400 may overlay real-time fluoroscopy image 311 with the selected vessel roadmap image 211. Step 460 is performed by vessel roadmap overlay 381.

In step 470, method 400 aligns the selected vessel roadmap image 211 and real-time fluoroscopy image 311 based on second alignment data 213 and real time second fluoroscopy information 313. Step 470 may include a sub-step 471, in which method 400 may align the position of the contrast application object with the position of the fluoroscopy object. Both step 470 and 471 are performed by vessel roadmap alignment 382.

In step 480, method 400 provides guidance for the fluoroscopy object to the pathological vessel based on the selected vessel roadmap 210 and the second fluoroscopy information 313. Step 480 may include a sub-step 481, in which method 400 may determine a path from the fluoroscopy object to the pathological vessel based on the second real-time fluoroscopy information 313 and the vessel segmentation data. Steps 480 and 481 are performed by pathological vessel guidance 383.

In step 490, method 400 may display the path to the pathological vessel on display 860 of medical imaging system 800 to guide an operator or medical specialist operating the fluoroscopy object to the pathological vessel. Step 490 may be performed by pathological vessel guidance 383.

As briefly discussed above, Figs. 8A and 8B show exemplary medical imaging system 800. Medical imaging system 800 may be used for both angiography and fluoroscopy. However, angiography and fluoroscopy may also be performed on separate systems with largely identical elements. In Fig. 8A, medical imaging system 800 is in neutral position P₀. In Fig. 8B, medical imaging system 800 is in a rotated position P₁. As discussed above, the angle between the two positions is referred to as the angiography angle or the fluoroscopy angle, depending on the imaging process currently performed by medical imaging system 800. Medical imaging system 800 includes C arm 830, on which X-ray emission means 831 and X-ray detection means 832 may be mounted. C arm 830 and thereby X-ray emission means 831 and X-ray detection means 832 are positioned to center around patient surface 840. X-ray emission means 831 may emit X-rays which may penetrate through a patient positioned on patient surface 840. X-ray detection means 832 detects the X -rays emitted from X-ray emission means 831. When a patient on patient surface 840 is injected with a radio-opaque contrast agent into the patient's vessels, some of the X-rays emitted by X-ray emission means 831 are absorbed by the radio-opaque contrast agent, leading X-ray detection means 832 to detect an image of the vessels filled with the radio-opaque contrast agent, i.e. an angiogram. X-ray emission means 831 and X-ray detection means 832 may also collectively be referred to as x-ray imaging means.

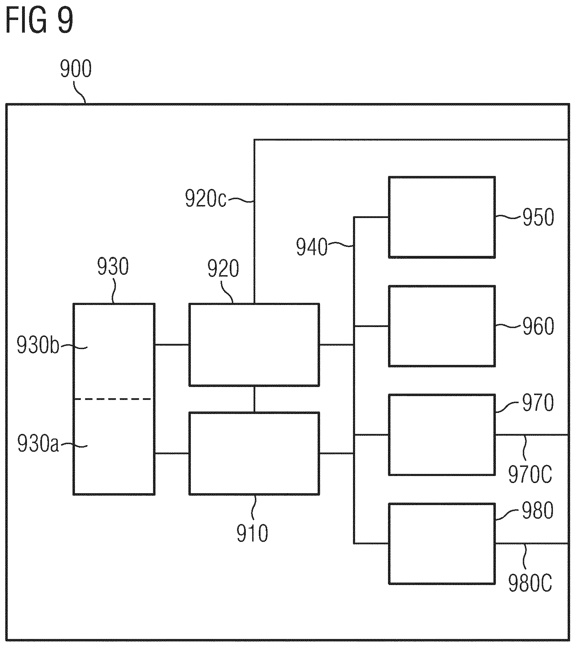

C arm 830 may be coupled to C arm rotation unit 820. C arm rotation unit 820 may be any motorized means configured to rotate C arm 830 according to an angiography angel or a fluoroscopy angle as either instructed by the medical specialist or angle comparison 380. C arm rotation unit 820 may be attached to and controlled by C arm control until 810. C arm control unit 810 may be any kind of circuitry capable of controlling C arm 830. For example, C arm control unit 810 may include computing device 900 of Fig. 9 or may be configured to interface with computing device 800.

Medical imaging system 800 may further include a control panel 850 mounted onto a side surface of patient surface support 841. Control panel 850 may be used to control C arm 830 in embodiments of the present invention in which method 400 displays real-time fluoroscopy image 311 with an overlaid vessel roadmap image 211 including the path to the one or more pathological vessels to the medical specialist in order to guide the medical specialist to the one or more pathological vessels. Fig. 8 does not show any connections between control panel 850 and C arm 830 to simplify the depiction of exemplary medical imaging system 800. In some embodiments, the connection may be wireless. In some embodiments, the connection may be wired and may e.g., be integrated into the ceiling of the room where medical imaging system 800 is located.

Medical imaging system 800 may finally also include a display 860. Display 860 may be used to display information to the medical specialist, such as real-time fluoroscopy image 311 with an overlaid vessel roadmap image 211 including the path to the one or more pathological vessels. Further, display 860 may be used to display the vessel segmentation data included in overlaid vessel roadmap image 211, including labels for the various vessel segments visible on display 360. In some embodiments, display 860 may be a touch screen, which may be used to toggle the display of the vessel segmentation data on and off. In some embodiments, display 860 may further display a confidence level indicating the confidence of roadmap selection 370, vessel comparison 381 and vessel roadmap alignment 382 in the accuracy of the overly and the alignment. In some embodiments, display 860 may also display, to the medical specialist, the appropriate angular position of C arm 830 during fluoroscopy to enable proper overlay and alignment of vessel roadmap image 211 as determined by angle comparison 380.

Fig. 9 shows a computing device 900 configured to perform method 400. Computing device 900 may include a processor 910, a graphics processing unit (GPU) 920, a memory 930, a bus 940, a storage 950, a removable storage 960, an medical imaging system control interface 970 and a communications interface 980.

Processor 910 may be any kind of single-core or multi-core processing unit employing a reduced instruction set (RISC) or a complex instruction set (CISC). Exemplary RISC processing units include ARM based cores or RISC V based cores. Exemplary CISC processing units include x86 based cores or x86-64 based cores. Processor 910 may further be an application specific integrated circuit (ASIC) or a field-programmable gate-array specially tailored to or programmed, respectively, to perform workflow 100 and method 400. Processor 410 may perform instructions causing computing device 400 to perform workflow 100 and method 400. Processor 910 may be directly coupled to any of the components of computing device 900 or may be directly coupled to memory 930, GPU 920 and bus 940.

GPU 920 may be any kind of processing unit optimized for processing graphics related instructions or more generally for parallel processing of instructions. As such, GPU 920 may perform part or all of method 400 to enable fast parallel processing of instructions relating to method 400. It should be noted that in some embodiments, processor 910 may determine that GPU 920 need not perform instructions relating to method 900. GPU 920 may be directly coupled to any of the components of computing device 900 or may be directly coupled to processor 910 and memory 930. GPU 920 may also be coupled to a display, such as display 860 of medical imaging system 800, via connection 920C. In some embodiments, GPU 920 may also be coupled to bus 940.

Memory 930 may be any kind of fast storage enabling processor 910 and GPU 920 to store instructions for fast retrieval during processing of the instructions well as to cache and buffer data. Memory 930 may be a unified memory coupled to both processor 910 and GPU 920 enabling allocation of memory 930 to processor 910 and GPU 920 as needed. Alternatively, processor 910 and GPU 920 may be coupled to separate processor memory 930a and GPU memory 930b.

Storage 950 may be a storage device enabling storage of program instructions and other data. For example, storage 950 may be a hard disk drive (HDD), a solid state disk (SSD) or some other type of non-volatile memory. Storage 950 may for example store the instructions of method 400 as well as the e.g. vessel image sequence 110 and vessel roadmap library 200.

Removable storage 960 may be a storage device which can be removably coupled with computing device 900. Examples include a digital versatile disc (DVD), a compact disc (CD), a Universal Serial Bus (USB) storage device, such as an external SSD, or a magnetic tape. Removable storage 940 may for example be used to provide the vessel image sequence 110 to computing device 900 and thereby to method 100 or to store the generated vessel roadmap library 200. It should be noted that removable storage 960 may also store other data, such as instructions of method 400, or may be omitted.

Storage 950 and removable storage 960 may be coupled to processor 910 via bus 940. Bus 940 may be any kind of bus system enabling processor 910 and optionally GPU 920 to communicate with storage device 950 and removable storage 960. Bus 940 may for example be a Peripheral Component Interconnect express (PCIe) bus or a Serial AT Attachment (SATA) bus.

Medical imaging system control interface 970 may enable computing device 900 to interface with medical imaging system 800 via connection 870C to control C arm 830 in accordance with method 400. For example, medical imaging system control interface 970 may be dedicated logic circuitry configured to control rotation of C arm 830. In some embodiments, medical imaging system control interface 470 may be C arm control unit 810. In some embodiments, medical imaging system control interface 870 may also be omitted and computing device 900 interfaces with medical imaging system 800 solely via communications interface 980. In such embodiments, processor 910 may control C arm directly via communications interface 970.

Communications interface 980 may enable computing device 900 to interface with external devices, either directly or via network, via connection 980C. Communications interface 980 may for example enable computing device 900 to couple to a wired or wireless network, such as Ethernet, Wifi, a Controller Area Network (CAN) bus or any bus system appropriate in medical systems. For example, computing device 900 may be coupled with medical imaging system 800 via connection 980C in order to receive vessel image sequence 110 or real-time fluoroscopy image 311 or to provide, overlay and align a selected vessel roadmap image 211. Communications interface may also be a USB port or a serial port to enable direct communication with an external device.

As stated above, computing device 900 may be integrated with medical imaging system 800. For example, computing device 900 may be integrated with C arm control unit 830 or may be placed inside patient surface support 840.

The disclosure may further be illustrated by the following examples.

In an example, a computer-implemented pathological vessel guidance method, comprises the steps of generating a vessel roadmap library, the vessel roadmap library including a plurality of vessel roadmaps, wherein each vessel roadmap comprises a vessel roadmap image and first and second alignment data, detecting, within the vessel roadmap images of the vessel roadmap library, a pathological vessel, obtaining a real-time fluoroscopy image and corresponding real-time first and second fluoroscopy information, selecting a vessel roadmap from the roadmap library based on comparing the first real-time fluoroscopy information with the first alignment data of each vessel roadmap of the roadmap library, overlaying the real-time fluoroscopy image with the selected vessel roadmap image of the vessel roadmap library, aligning the vessel roadmap image of the selected vessel roadmap and the real-time fluoroscopy image based on the second alignment data and the real time second fluoroscopy information; and providing guidance for a fluoroscopy object to the pathological vessel based on the selected vessel roadmap and the second fluoroscopy information.

In an example, detecting the pathological vessel may include determining centerlines of the vessels included in each vessel roadmap image of the vessel roadmap library, determining, based on the centerlines, lumina of the vessels included in each vessel roadmap image of the vessel roadmap library and detecting the pathological vessel based on the lumina of the vessels included in each vessel roadmap image of the vessel roadmap library.

In an example, generating a vessel roadmap library may include recording, for each vessel roadmap image of the vessel roadmap library, one or more imaging parameters, the imaging parameters indicating one or more parameters associated with an imaging method used to obtain the vessel roadmap image.

In an example, the one or more imaging parameters may include at least one of an angiography angle used to obtain each vessel roadmap image and a contrast medium dosage used to obtain each vessel roadmap image.

In an example, the one or more imaging parameters may include at least an angiography angle used to obtain each vessel roadmap image and the method may include comparing the angiography angle with a fluoroscopy angle, the fluoroscopy angle being used to obtain the real-time fluoroscopy image, and, if the angiography angle and the fluoroscopy angle differ by more than an angle difference threshold, the method refrains from overlaying the real-time fluoroscopy image with the selected vessel roadmap image.

In an example, providing guidance for the fluoroscopy object to the pathological vessel may include determining a path from the fluoroscopy object to the pathological vessel based on the second real-time fluoroscopy information and the vessel segmentation data.

In an example, the method may further comprise the step of displaying the path on a display of a medical imaging system to guide an operator operating the fluoroscopy object to the pathological vessel.

In an example, the second alignment data may be derived from the corresponding vessel roadmap image.

In an example, generating the vessel roadmap may further include obtaining a vessel image sequence using an imaging method based on an in-flow of a contrast medium into a vessel tree via a contrast application object and im-aging physiological information associated with the vessel image sequence, detecting, within the vessel image sequence, contrasted vessel images, performing vessel segmentation on the contrasted vessel images to generate vessel segmentation data, performing contrast application object segmentation on the contrasted vessel images to generate contrast application object segmentation data identifying a posi-tion of the contrast application object in the contrasted vessel images; and for each contrasted vessel image, generating a vessel roadmap, the generated vessel roadmap comprising the contrasted vessel image and the vessel segmentation data as the vessel roadmap image, the imaging physiological information in the first alignment data and the contrast application object segmentation data in the second alignment data.

In an example, the imaging physiological information may include an electrocardiogram (ECG) and generating the vessel roadmap may further include identifying one or more cardiac cycles within the vessel image se-quence based on the ECG, wherein the generated vessel roadmap further comprises the identified one or more cardiac cycles in the first alignment data.

In an example, obtaining a real-time fluoroscopy image and corresponding real-time first and second fluoroscopy information may include obtaining fluoroscopy physiological information associated with the fluoroscopy image and performing fluoroscopy object segmentation on the fluoroscopy image to generate fluoroscopy object segmentation data identifying a position of the fluoroscopy object in the fluoroscopy image, wherein the fluoroscopy physiological information may be included in the first real-time fluoroscopy information, and wherein the generated fluoroscopy object segmentation data may be included in the second real-time fluoroscopy information.

In an example, the fluoroscopy physiological information may include an electrocardiogram (ECG) and wherein obtaining the real-time fluoroscopy image and corresponding real-time first and second fluoroscopy information may further comprise identifying one or more cardiac cycles based on the ECG, wherein the identified one or more cardiac cycles are included in the first real-time fluoroscopy information.

In an example, aligning the vessel roadmap image and the real-time fluoroscopy image based on the second alignment data and the real time second fluoroscopy information may include aligning the position of the contrast application object with the position of the fluoroscopy object.

In an example, a computer-readable medium comprising instructions configured to be executed by a computer including at least one processor, the instructions causing the processor to perform the method according to any one of the preceding examples.

The preceding description has been provided to illustrate how to guide a fluoroscopy object to pathological vessel based on dynamic vessel roadmaps. It should be understood that the description is in no way meant to limit the scope of the invention to the precise embodiments discussed throughout the description.

## Claims

1. Computer-implemented method for providing an operator with guidance to a pathological vessel (400) comprising the steps of:
generating (410) a vessel roadmap library (200), the vessel roadmap library (200) including a plurality of vessel roadmaps (210), wherein each vessel roadmap (210) comprises a vessel roadmap image (211) and first and second alignment data (212, 213);
detecting (420), within the vessel roadmap images (211) of the vessel roadmap library (210), a pathological vessel; obtaining (430) a real-time fluoroscopy image (311) and corresponding real-time first fluoroscopy information (312) and real-time second fluoroscopy information (313);
selecting (440) a vessel roadmap (210) from the roadmap library (200) based on comparing the real-time first fluoroscopy information (312) with the first alignment data (212) of each vessel roadmap (210) of the roadmap library (200);
overlaying (460) the real-time fluoroscopy image (311) with the selected vessel roadmap image (211) of the vessel roadmap library (200);
aligning (470) the vessel roadmap image of the selected vessel roadmap and the real-time fluoroscopy image based on the second alignment data and the real-time second fluoroscopy information; and
providing an operator with guidance for a fluoroscopy object to the pathological vessel based on the selected vessel roadmap and the real-time second fluoroscopy information.

2. The method (400) of claim 1, wherein detecting (420) the pathological vessel includes:
determining (421) centerlines of the vessels included in each vessel roadmap image (211) of the vessel roadmap library (200);
determining (422), based on the centerlines, lumina of the vessels included in each vessel roadmap image (211) of the vessel roadmap library (200); and
detecting (423) the pathological vessel based on the lumina of the vessels included in each vessel roadmap image (211) of the vessel roadmap library (200).

3. The method (400) of any one of claims 1 and 2, wherein generating (410) the vessel roadmap library (200) includes:
recording (411), for each vessel roadmap image (211) of the vessel roadmap library (200), one or more imaging parameters (214), the imaging parameters indicating one or more parameters associated with an imaging method used to obtain the vessel roadmap image (211).

4. The method of claim 3, wherein the one or more imaging parameters (214) include at least one of an angiography angle (214a) and a contrast medium dosage (214b).

5. The method (400) of claim 3, wherein
the one or more imaging parameters (214) includes at least an angiography angle (214a) used to obtain each vessel roadmap image;
the method includes comparing (450) the angiography angle (214a) with a fluoroscopy angle (314b), the fluoroscopy angle being used to obtain the real-time fluoroscopy image (311); and
if the angiography angle (214a) and the fluoroscopy angle (314a) differ by more than an angle difference threshold, the method refrains from overlaying (460) the real-time fluoroscopy image (311) with the selected vessel roadmap image (211).

6. The method (400) of any one of the preceding claims, wherein providing guidance for the fluoroscopy object to the pathological vessel includes determining a path from the fluoroscopy object to the pathological vessel based on the second real-time fluoroscopy information (313) and vessel segmentation data.

7. The method (400) of claim 6, further comprising the step of:
displaying (490) the path on a display of a medical imaging system (800) to guide an operator operating the fluoroscopy object to the pathological vessel.

8. The method (400) of any one of the preceding claims, wherein the second alignment data (213) is derived from the corresponding vessel roadmap image (211).

9. The method (400) of any one of the preceding claims, wherein generating (410) the vessel roadmap further includes:
obtaining (412) a vessel image sequence (110) using an imaging method based on an inflow of a contrast medium into a vessel tree via a contrast application object and imaging physiological information (111) associated with the vessel image sequence;
detecting (413), within the vessel image sequence (110), contrasted vessel images;
performing (414) vessel segmentation on the contrasted vessel images to generate vessel segmentation data;
performing (415) contrast application object segmentation on the contrasted vessel images to generate contrast application object segmentation data identifying a position of the contrast application object in the contrasted vessel images; and
for each contrasted vessel image, generating (416) a vessel roadmap (210), the generated vessel roadmap comprising:
the contrasted vessel image and the vessel segmentation data as the vessel roadmap image (211); the imaging physiological information in the first alignment data (212); and
the contrast application object segmentation data in the second alignment data (213).

10. The method (400) of claim 9, wherein:
the imaging physiological information includes an electrocardiogram -ECG-, and
generating (416) the vessel roadmap (210) further includes:
identifying (417) one or more cardiac cycles within the vessel image sequence (110) based on the ECG, wherein the generated vessel roadmap (210) further comprises the identified one or more cardiac cycles in the first alignment data (211).

11. The method (400) of any one of the preceding claims, wherein obtaining (430) a real-time fluoroscopy image (311) and corresponding real-time first fluoroscopy information (312) and real-time second fluoroscopy information (313) includes:
obtaining (431) fluoroscopy physiological information associated with the fluoroscopy image (311); and
performing (432) fluoroscopy object segmentation on the fluoroscopy image (311) to generate fluoroscopy object segmentation data identifying a position of the fluoroscopy object in the fluoroscopy image (311),
wherein the fluoroscopy physiological information is included in the first real-time fluoroscopy information (312), and
wherein the generated fluoroscopy object segmentation data is included in the second real-time fluoroscopy information (313).

12. The method (400) of claim 11, wherein the fluoroscopy physiological information includes an electrocardiogram -ECG- and wherein obtaining the real-time fluoroscopy image (311) and corresponding real-time first fluoroscopy information (312) and real-time second fluoroscopy information (313) further comprises identifying (433) one or more cardiac cycles based on the ECG, wherein the identified one or more cardiac cycles are included in the first real-time fluoroscopy information (312).

13. The method (400) of any one of claims 11 to 12, wherein aligning (470) the vessel roadmap image (211) and the real-time fluoroscopy image (311) based on the second alignment data (213) and the real-time second fluoroscopy information (313) includes aligning (471) the position of the contrast application object with the position of the fluoroscopy object.

14. A computer-readable medium (950, 960) comprising instructions configured to be executed by a computer including at least one processor (910), the instructions causing the processor (910) to perform the method according to any one of claim 1 to 13.

## Patentansprüche

1. Computerimplementiertes Verfahren zum Bereitstellen einer Führung für einen Bediener zu einem pathologischen Gefäß (400), umfassend die Schritte:
Erzeugen (410) einer Gefäßüberblicksplanbibliothek (200), wobei die Gefäßüberblicksplanbibliothek (200) eine Vielzahl von Gefäßüberblicksplänen (210) umfasst, wobei jeder Gefäßüberblicksplan (210) ein Gefäßüberblicksplanbild (211) sowie erste und zweite Ausrichtungsdaten (212, 213) beinhaltet;
Erkennen (420) eines pathologischen Gefäßes innerhalb der Gefäßübersichtsplanbilder (211) der Gefäßübersichtsplanbibliothek (210); Erfassen (430) eines Echtzeit-Fluoroskopiebildes (311) sowie entsprechender erster Echtzeit-Fluoroskopieinformationen (312) und zweiter Echtzeit-Fluoroskopieinformationen (313);
Auswählen (440) eines Gefäßübersichtsplans (210) aus der Übersichtsplanbibliothek (200) basierend auf einem Vergleich der ersten Echtzeit-Fluoroskopieinformationen (312) mit den ersten Ausrichtungsdaten (212) jedes Gefäßübersichtsplans (210) der Übersichtsplanbibliothek (200);
Überlagern (460) des Echtzeit-Fluoroskopiebildes (311) mit dem ausgewählten Gefäßübersichtsplanbild (211) der Gefäßübersichtsplanbibliothek (200);
Ausrichten (470) des Gefäßübersichtsplanbildes des ausgewählten Gefäßübersichtsplans und des Echtzeit-Fluoroskopiebildes basierend auf den zweiten Ausrichtungsdaten und den zweiten Echtzeit-Fluoroskopieinformationen; und
Bereitstellen einer Führung für den Bediener zu einem Fluoroskopieobjekt zum pathologischen Gefäß basierend auf dem ausgewählten Gefäßübersichtsplan und den zweiten Echtzeit-Fluoroskopieinformationen.

2. Verfahren (400) nach Anspruch 1, wobei das Erkennen (420) des pathologischen Gefäßes umfasst:
Bestimmen (421) von Mittellinien der Gefäße, die in jedem Gefäßübersichtsplanbild (211) der Gefäßübersichtsplanbibliothek (200) enthalten sind;
Bestimmen (422) der Lumina der Gefäße, die in jedem Gefäßübersichtsplanbild (211) der Gefäßübersichtsplanbibliothek (200) enthalten sind, basierend auf den Mittellinien; und
Erkennen (423) des pathologischen Gefäßes basierend auf den Lumina der Gefäße, die in jedem Gefäßübersichtsplanbild (211) der Gefäßübersichtsplanbibliothek (200) enthalten sind.

3. Verfahren (400) nach einem der Ansprüche 1 oder 2, wobei das Erzeugen (410) der Gefäßübersichtsplanbibliothek (200) beinhaltet:
Aufzeichnen (411) eines oder mehrerer Bildgebungsparameter (214) für jedes Gefäßübersichtsplanbild (211) der Gefäßübersichtsplanbibliothek (200), wobei die Bildgebungsparameter einen oder mehrere Parameter angeben, die einem Bildgebungsverfahren zugeordnet sind, das zum Erhalten des Gefäßübersichtsplanbilds (211) verwendet wurde.

4. Verfahren nach Anspruch 3,
wobei der eine oder die mehreren Bildgebungsparameter (214) einen Angiographiewinkel (214a) und/oder eine Kontrastmitteldosis (214b) beinhalten.

5. Verfahren (400) nach Anspruch 3, wobei der eine oder die mehreren Bildgebungsparameter (214) mindestens einen Angiographiewinkel (214a) beinhalten, der zum Erhalten jedes Gefäßübersichtsplanbildes verwendet wird;
das Verfahren das Vergleichen (450) des Angiographiewinkels (214a) mit einem Fluoroskopiewinkel (314b) beinhaltet, wobei der Fluoroskopiewinkel zum Erhalten des Echtzeit-Fluoroskopiebildes (311) verwendet wird; und
wenn sich der Angiographiewinkel (214a) und der Fluoroskopiewinkel (314a) um mehr als eine Winkeldifferenzschwelle unterscheiden, das Verfahren darauf verzichtet, das Echtzeit-Fluoroskopiebild (311) mit dem ausgewählten Gefäßübersichtsplanbild (211) zu überlagern (460).

6. Verfahren (400) nach einem der vorhergehenden Ansprüche, wobei das Bereitstellen einer Führung für das Fluoroskopieobjekt zu dem pathologischen Gefäß das Bestimmen eines Weges von dem Fluoroskopieobjekt zu dem pathologischen Gefäß basierend auf den zweiten Echtzeit-Fluoroskopieinformationen (313) und Gefäßsegmentierungsdaten beinhaltet.

7. Verfahren (400) nach Anspruch 6, ferner umfassend den folgenden Schritt:
Anzeigen (490) des Weges auf einer Anzeigevorrichtung eines medizinischen Bildgebungssystems (800), um einen Bediener, der das Fluoroskopieobjekt bedient, zu dem pathologischen Gefäß zu führen.

8. Verfahren (400) nach einem der vorhergehenden Ansprüche, wobei die zweiten Ausrichtungsdaten (213) aus dem entsprechenden Gefäßübersichtsplanbild (211) abgeleitet werden.

9. Verfahren (400) nach einem der vorhergehenden Ansprüche, wobei das Erzeugen (410) des Gefäßübersichtsplans ferner beinhaltet:
Erhalten (412) einer Gefäßbildsequenz (110) unter Verwendung eines Bildgebungsverfahrens basierend auf einem Einströmen eines Kontrastmediums in einen Gefäßbaum über ein Kontrastanwendungsobjekt und bildgebende physiologische Informationen (111), die der Gefäßbildsequenz zugeordnet sind;
Erfassen (413) von kontrastierten Gefäßbildern innerhalb der Gefäßbildsequenz (110);
Durchführen (414) einer Gefäßsegmentierung an den kontrastierten Gefäßbildern, um Gefäßsegmentierungsdaten zu erzeugen;
Durchführen (415) einer Kontrastanwendungsobjektsegmentierung an den kontrastierten Gefäßbildern, um Kontrastanwendungsobjektsegmentierungsdaten zu erzeugen, die ein Gefäßbild identifizieren; und
Erzeugen (416) eines Gefäßübersichtsplans (210) für jedes kontrastierte Gefäßbild, wobei der erzeugte Gefäßübersichtsplan umfasst:
das kontrastierte Gefäßbild und die Gefäßsegmentierungsdaten als das Gefäßübersichtsplanbild (211); die bildgebenden physiologischen Informationen in den ersten Ausrichtungsdaten (212); und
die Kontrastanwendungsobjektsegmentierungsdaten in den zweiten Ausrichtungsdaten (213).

10. Verfahren (400) nach Anspruch 9, wobei
die bildgebenden physiologischen Informationen ein Elektrokardiogramm, EKG, beinhalten und das Erzeugen (416) des Gefäßübersichtsplans (210) ferner beinhaltet:
Identifizieren (417) eines oder mehrerer Herzzyklen innerhalb der Gefäßbildsequenz (110) basierend auf dem EKG, wobei der erzeugte Gefäßübersichtsplan (210) ferner den identifizierten einen oder die identifizierten mehreren Herzzyklen in den ersten Ausrichtungsdaten (211) umfasst.

11. Verfahren (400) nach einem der vorhergehenden Ansprüche, wobei das Erhalten (430) eines Echtzeit-Fluoroskopiebildes (311) und entsprechender erster Echtzeit-Fluoroskopieinformationen (312) und zweiter Echtzeit-Fluoroskopieinformationen (313) beinhaltet:
Erhalten (431) von physiologischen Fluoroskopieinformationen, die dem Fluoroskopiebild (311) zugeordnet sind; und
Durchführen (432) einer Fluoroskopieobjektsegmentierung an dem Fluoroskopiebild (311), um Fluoroskopieobjektsegmentierungsdaten zu erzeugen, die eine Position des Fluoroskopieobjekts in dem Fluoroskopiebild (311) identifizieren,
wobei die physiologischen Fluoroskopieinformationen in den ersten Echtzeit-Fluoroskopieinformationen (312) enthalten sind, und
wobei die erzeugten Fluoroskopieobjektsegmentierungsdaten in den zweiten Echtzeit-Fluoroskopieinformationen enthalten sind (313).

12. Verfahren (400) nach Anspruch 11, wobei die physiologischen Fluoroskopieinformationen ein Elektrokardiogramm, EKG, beinhalten und wobei das Erhalten des Echtzeit-Fluoroskopiebilds (311) und entsprechender erster Echtzeit-Fluoroskopieinformationen (312) und zweiter Echtzeit-Fluoroskopieinformationen (313) ferner das Identifizieren (433) eines oder mehrerer Herzzyklen basierend auf dem EKG umfasst, wobei der eine oder die mehreren identifizierten Herzzyklen in den ersten Echtzeit-Fluoroskopieinformationen (312) enthalten sind.

13. Verfahren (400) nach einem der Ansprüche 11 bis 12, wobei das Ausrichten (470) des Gefäßübersichtsplanbilds (211) und des Echtzeitfluoroskopiebilds (311) basierend auf den zweiten Ausrichtungsdaten (213) und den zweiten Echtzeit-Fluoroskopieinformationen (313) das Ausrichten (471) der Position des Kontrastanwendungsobjekts mit der Position des Fluoroskopieobjekts beinhaltet.

14. Computerlesbares Medium (950, 960), das Anweisungen umfasst, die dazu ausgelegt sind, durch einen Computer ausgeführt zu werden, der mindestens einen Prozessor (910) beinhaltet, wobei die Anweisungen bewirken, dass der Prozessor (910) das Verfahren nach einem der Ansprüche 1 bis 13 durchführt.

## Revendications

1. Procédé mis en œuvre par ordinateur pour fournir à un opérateur un guidage jusqu'à un vaisseau pathologique (400), comprenant les étapes suivantes :
la génération (410) d'une bibliothèque de cartes de vaisseaux (200), la bibliothèque de cartes de vaisseaux (200) incluant une pluralité de cartes de vaisseaux (210), dans lequel chaque carte de vaisseaux (210) comprend une image de carte de vaisseaux (211) et des premières et secondes données d'alignement (212, 213) ;
la détection (420), dans les images de carte de vaisseaux (211) de la bibliothèque de cartes de vaisseaux (210), d'un vaisseau pathologique ; l'obtention (430) d'une image par fluoroscopie en temps réel (311) et de premières informations de fluoroscopie en temps réel (312) et de secondes informations de fluoroscopie en temps réel (313) correspondantes ;
la sélection (440) d'une carte de vaisseaux (210) parmi la bibliothèque de cartes (200) sur la base d'une comparaison des premières informations de fluoroscopie en temps réel (312) avec les premières données d'alignement (212) de chaque carte de vaisseaux (210) de la bibliothèque de cartes (200) ;
la superposition (460) de l'image par fluoroscopie en temps réel (311) avec l'image de carte de vaisseaux (211) sélectionnée de la bibliothèque de cartes de vaisseaux (200) ;
l'alignement (470) de l'image de carte de vaisseaux de la carte de vaisseaux sélectionnée et de l'image par fluoroscopie en temps réel sur la base des secondes données d'alignement et des secondes informations de fluoroscopie en temps réel ; et
la fourniture à un opérateur d'un guidage pour un objet fluoroscopique jusqu'au vaisseau pathologique sur la base de la carte de vaisseaux sélectionnée et des secondes informations de fluoroscopie en temps réel.

2. Procédé (400) selon la revendication 1, dans lequel la détection (420) du vaisseau pathologique inclut :
la détermination (421) de lignes centrales des vaisseaux inclus dans chaque image de carte de vaisseaux (211) de la bibliothèque de cartes de vaisseaux (200) ;
la détermination (422), sur la base des lignes centrales, de lumières des vaisseaux inclus dans chaque image de carte de vaisseaux (211) de la bibliothèque de cartes de vaisseaux (200) ; et
la détection (423) du vaisseau pathologique sur la base des lumières des vaisseaux inclus dans chaque image de carte de vaisseaux (211) de la bibliothèque de cartes de vaisseaux (200).

3. Procédé (400) selon l'une quelconque des revendications 1 et 2, dans lequel la génération (410) de la bibliothèque de cartes de vaisseaux (200) inclut :
l'enregistrement (411), pour chaque image de carte de vaisseaux (211) de la bibliothèque de cartes de vaisseaux (200), d'un ou plusieurs paramètres d'imagerie (214), les paramètres d'imagerie indiquant un ou plusieurs paramètres associés à un procédé d'imagerie utilisé pour obtenir l'image de carte de vaisseaux (211).

4. Procédé selon la revendication 3,
dans lequel les un ou plusieurs paramètres d'imagerie (214) incluent au moins l'un d'un angle angiographique (214a) et d'un dosage de produit de contraste (214b).

5. Procédé (400) selon la revendication 3, dans lequel les un ou plusieurs paramètres d'imagerie (214) incluent au moins un angle angiographique (214a) utilisé pour obtenir chaque image de carte de vaisseaux ;
le procédé inclut la comparaison (450) de l'angle angiographique (214a) avec un angle fluoroscopique (314b), l'angle fluoroscopique étant utilisé pour obtenir l'image fluoroscopique en temps réel (311) ; et
si l'angle angiographique (214a) et l'angle fluoroscopique (314a) diffèrent de plus d'un seuil de différence d'angle, le procédé s'abstient de superposer (460) l'image par fluoroscopie en temps réel (311) avec l'image de carte de vaisseaux (211) sélectionnée.

6. Procédé (400) selon l'une quelconque des revendications précédentes, dans lequel la fourniture d'un guidage pour l'objet fluoroscopique jusqu'au vaisseau pathologique inclut la détermination d'un trajet de l'objet fluoroscopique au vaisseau pathologique sur la base des secondes informations de fluoroscopie en temps réel (313) et de données de segmentation de vaisseau.

7. Procédé (400) selon la revendication 6, comprenant en outre l'étape suivante :
l'affichage (490) du trajet sur un écran d'un système d'imagerie médicale (800) pour guider un opérateur utilisant l'objet fluoroscopique jusqu'au vaisseau pathologique.

8. Procédé (400) selon l'une quelconque des revendications précédentes, dans lequel les secondes données d'alignement (213) sont dérivées de l'image de carte de vaisseaux (211) correspondante.

9. Procédé (400) selon l'une quelconque des revendications précédentes, dans lequel la génération (410) de la carte de vaisseaux inclut en outre :
l'obtention (412) d'une séquence d'images de vaisseau (110) à l'aide d'un procédé d'imagerie basé sur un flux entrant d'un produit de contraste dans un arbre de vaisseaux par l'intermédiaire d'un objet d'application de contraste et d'informations physiologiques d'imagerie (111) associées à la séquence d'images de vaisseau ;
la détection (413), dans la séquence d'images de vaisseau (110), d'images de vaisseau contrastées ;
la réalisation (414) d'une segmentation de vaisseau sur les images de vaisseau contrastées pour générer des données de segmentation de vaisseau ;
la réalisation (415) d'une segmentation d'objet d'application de contraste sur les images de vaisseau contrastées pour générer des données de segmentation d'objet d'application de contraste identifiant des images de vaisseau ; et
pour chaque image de vaisseau contrastée, la génération (416) d'une carte de vaisseaux (210), la carte de vaisseaux générée comprenant :
l'image de vaisseau contrastée et les données de segmentation de vaisseau en tant qu'image de carte de vaisseaux (211) ; les informations physiologiques d'imagerie dans les premières données d'alignement (212) ; et
les données de segmentation d'objet d'application de contraste dans les secondes données d'alignement (213).

10. Procédé (400) selon la revendication 9, dans lequel :
les informations physiologiques d'imagerie incluent un électrocardiogramme, ECG, et la génération (416) de la carte de vaisseaux (210) inclut en outre :
l'identification (417) d'un ou plusieurs cycles cardiaques dans la séquence d'images de vaisseau (110) sur la base de l'ECG, dans lequel la carte de vaisseaux (210) générée comprend en outre les un ou plusieurs cycles cardiaques identifiés dans les premières données d'alignement (211).

11. Procédé (400) selon l'une quelconque des revendications précédentes, dans lequel l'obtention (430) d'une image par fluoroscopie en temps réel (311) et de premières informations de fluoroscopie en temps réel (312) et de secondes informations de fluoroscopie en temps réel (313) correspondantes inclut :
l'obtention (431) d'informations physiologiques de fluoroscopie associées à l'image par fluoroscopie (311) ; et
la réalisation (432) d'une segmentation d'objet fluoroscopique sur l'image par fluoroscopie (311) pour générer des données de segmentation d'objet fluoroscopique identifiant une position de l'objet fluoroscopique dans l'image par fluoroscopie (311),
dans lequel les informations physiologiques de fluoroscopie sont incluses dans les premières informations de fluoroscopie en temps réel (312), et
dans lequel les données de segmentation d'objet fluoroscopique générées sont incluses dans les secondes informations de fluoroscopie en temps réel (313).

12. Procédé (400) selon la revendication 11, dans lequel les informations physiologiques de fluoroscopie incluent un électrocardiogramme, ECG, et dans lequel l'obtention de l'image par fluoroscopie en temps réel (311) et de premières informations de fluoroscopie en temps réel (312) et de secondes informations de fluoroscopie en temps réel (313) correspondantes comprend en outre l'identification (433) d'un ou plusieurs cycles cardiaques sur la base de l'ECG, dans lequel les un ou plusieurs cycles cardiaques identifiés sont inclus dans les premières informations de fluoroscopie en temps réel (312).

13. Procédé (400) selon l'une quelconque des revendications 11 à 12, dans lequel l'alignement (470) de l'image de carte de vaisseaux (211) et de l'image par fluoroscopie en temps réel (311) sur la base des secondes données d'alignement (213) et des secondes informations de fluoroscopie en temps réel (313) inclut l'alignement (471) de la position de l'objet d'application de contraste avec la position de l'objet fluoroscopique.

14. Support lisible par ordinateur (950, 960) comprenant des instructions configurées pour être exécutées par un ordinateur incluant au moins un processeur (910), les instructions amenant le processeur (910) à réaliser le procédé selon l'une quelconque des revendications 1 à 13.
